(19) **Europäisches Patentamt**

**European Patent Office**

**Office européen des brevets**

(11) Veröffentlichungsnummer : **0 341 198 B1**

(12) ## EUROPÄISCHE PATENTSCHRIFT

(45) Veröffentlichungstag der Patentschrift :
**03.06.92 Patentblatt 92/23**

(21) Anmeldenummer : **89810165.4**

(22) Anmeldetag : **03.03.89**

(51) Int. Cl.⁵ : **A61F 2/34**

(54) **Künstliche Hüftgelenkspfanne.**

(30) Priorität : **11.04.88 CH 1322/88**

(43) Veröffentlichungstag der Anmeldung :
**08.11.89 Patentblatt 89/45**

(45) Bekanntmachung des Hinweises auf die
Patenterteilung :
**03.06.92 Patentblatt 92/23**

(84) Benannte Vertragsstaaten :
**AT BE DE ES FR GB IT NL**

(56) Entgegenhaltungen :
**DE-A- 2 807 289**
**DE-A- 2 950 536**

(56) Entgegenhaltungen :
**BIOMEDIZINISCHE TECHNIK, Band 32, Nr. 3,
März 1987, Seiten 40-45, Berlin, DE; D. GE-
BAUER: "Die zementlose Hüftgelenkpfanne
-Analyse der Fixierungsbedingungen der unterschiedlichen Konstruktionsprinzipien"**

(73) Patentinhaber : **GEBRÜDER SULZER
AKTIENGESELLSCHAFT
Zürcherstrasse 9
CH-8401 Winterthur (CH)**
Patentinhaber : **ALLO PRO AG
Grabenstrasse 25
CH-6340 Baar (CH)**

(72) Erfinder : **Karpf, Kurt
Alte Strasse 175
CH-4718 Holderbank (CH)**

## Beschreibung

Die Erfindung betrifft eine künstliche Hüftgelenkspfanne für eine zementfreie Verankerung im Beckenknochen, welche Pfanne aus einer metallischen Aussenschale und einem Kunststoff-Pfannenkörper besteht, der die eigentliche Pfannenschale zur Aufnahme eines Gelenkkopfes enthält, wobei die Aussenschale die Form eines Kegelstumpfes hat, dessen Grundfläche im Scheitelbereich mindestens eine Oeffnung aufweist, wobei ferner die äussere Oberfläche der Aussenschale mit einer zirkulären Verzahnung versehen ist.

Eine derartige Konstruktion ist bekannt aus der EP-B-0 083 708. Die Aussenschale dieser Konstruktion ist geschlitzt und wird zur Fixation im Beckenknochen beim Einpressen des Pfannenkörpers aufgeweitet. Andere, sehr ähnliche Konstruktionen sind auf der Mantelfläche der kegelstumpfförmigen Aussenschale mit einen, häufig selbstschneidend ausgebildeten Gewinde versehen, mit dem sie in den Beckenknochen eingeschraubt werden.

Für den Einsatz derartige Hüftgelenkspfannen ist häufig insbesondere bei Reoperationen, bei denen eine neue Aussenschale eingesetzt werden muss - im Bereich des natürlichen Azetabulums nicht mehr genügend gesundes Knochenmaterial vorhanden, um eine sichere Verankerung zu gewährleisten. Darüberhinaus erfordert die Implantation der bekannten Konstruktionen vom Operateur sehr viel Erfahrung, da die Knochen bei den einzelnen Patienten verschieden hart sind, wodurch selbstverständlich das Aufspreizen oder das Eindrehen einer Aussenschale beeinflusst werden.

Aufgabe der Erfindung ist es daher, eine zementfreie zu verankernde Hüftgelenkspfanne zu schaffen, welche - primär und sekundär - stabil im Becken fixiert ist, für den Operateur während der Implantation leicht zu handhaben ist, und deren endgültiger Sitz im operativ vorbereiteten Knochen reproduzierbar und gleichmässig fest ist und durch den Operateur nicht mehr oder nur möglichst geringfügig beeinflusst werden kann.

Diese Aufgabe wird mit der Erfindung gelöst durch die Kombination der folgenden Merkmale:
- Die Verzahnung ist sägezahnartig ausgebildet und weist eine Zahntiefe von maximal 1 mm auf;
- aus dem Mantel des Kegelstumpfes ragen mindestens drei, ungleichmässig auf dem Umfang verteilte, als Spreizdübel ausgebildete Verankerungszapfen hervor, deren Achsen parallel zur Kegelstumpfachse verlaufen;
- die geschlitzte, spreizbare Höhe der Dübel ist auf ihren den Kegelstumpf überragenden Abschnitt beschränkt und trägt ebenfalls eine sägezahnartige, zirkuläre Verzah nung, während die Dübeloberfläche im Bereich der Kegel stumpfhöhe verzahnungsfrei ausgebildet ist;
- der Durchmesser eines die Dübel berührenden, mit dem Kegelstumpf konzentrischen Innenkreises beträgt mindestens 2/3 des basisseitigen Innendurchmessers des den Pfannenkörper aufnehmenden Innenhohlraumes der Aussen schale, während der Durchmesser des zugehörigen, die Dübel umschliessenden Aussenkreises kleiner ist als derjenige des basisseitigen Aussenrandes der Aussen schale.

Die sägezahnförmige Feinverzahnung und die vorgeschriebene geringe Zahntiefe ermöglichen ein Einwachsen von Knochensubstanz - und nicht wie häufig nur von einfachem Bindegewebe - bis auf den "Grund" der Zähne. Dadurch wird besonders die Stabilität der Sekundärverankerung gewährleistet. Diese lässt sich darüberhinaus weiter verbessern, wenn die Verzahnung der äusseren Oberfläche der Aussenschale mit entlang Mantellinien und Radien verlaufenden Rillen versehen wird.

Die drei- oder mehrzylindrischen Spreizdübel am Umfang der Aussenpfanne liegen genau in Einschubrichtung der Pfanne. Sie sind innerhalb des grössten Pfannendurchmessers angeordnet, jedoch möglichst weit aussen, damit sie sich mit grosser Wahrscheinlichkeit in gesundem Knochengewebe erstrecken.

Ihre Anordnung ist hinsichtlich einer Rotation um die Kegelstumpfachse ungleichmässig, damit die Aussenschale nur in einer vorausbestimmten Position eingesetzt werden kann. Dies ist for allem bei der Implantation von Pfannenkörpern mit erhöhtem Pfannendach wesentlich.

Innerhalb der Kegelstumphöhe sind die Dübel verzahnungsfrei glatt oder mit einer rauhen Oberflächenstrukturierung versehen, während ihr Spreizbereich in der über den Kegelstumpf hinausgehenden Verlängerung liegt. Dadurch können sie beim Einsetzen der Aussenschale in das operativ vorbereitete Becken in entsprechend vorgebohrten Bohrungen exakt geführt werden, wodurch auf einfache Weise das Einsetzen der Schale in der "richtigen" Richtung erleichtert wird.

Jegliches Eindrehen eines Schraubengewindes, dessen Anziehen individuell vom Operateur abhängig ist, kann vermieden werden, wenn als Spreizelement gewindefreie, in Längsrichtung eines Dübels einschlagbare, zylindrische Bolzen dienen, die an einem Ende mit einem durch eine Schulter abgesetzten Kopf und am anderen Ende mit einer durch eine zirkuläre Führungsrille erzeugten Kerbe ausgestattet sind; in die Kerbe kann das freie Ende des Spreizdübels einrasten und so als Sicherung gegen unbeabsichtigtes Herausrutschen des Spreizelementes dienen. Wird jedoch ein bewusstes Entfernen der Spreizelemente notwendig, wofür diese in ihrem Kopf mit einem Gewinde für den Ansatz eines Instrumentes versehen sein können, so kann das die Kerbe überragende, pilzkopfartige Ende des Spreizelements abgerissen weden.

Ist der Abstand zwischen der Schulter und der Kerbe des Spreizelementes gleich demjenigen eines Absatzes für die Auflage des Kopfes im inneren Hohlraum des Dübels und dessen freien Ende, so ergibt sich eine vom Einschlagen der Bolzen durch den Operateur unabhängige, immer gleiche Spreizkraft für alle Dübel.

Um ein Hinterfüllen der Aussenschale mit Knochenmaterial zu ermöglichen, sollte zweckmässigerweise die Fläche der Oeffnung im Scheitelbereich der Aussenschale mindestens 1/3 der Grundfläche des Kegelstumpf-Kopfkreises betragen. Direkte Kontakte zwischen Knochen und Kunststoff können dabei vermieden werden, wenn die Oeffnung durch einen metallischen Deckel verschliessbar ist.

Die Elastizität der Aussenschale lässt sich verbessern, wenn sie von ihren Rändern her mit Einschnitten versehen ist.

In besonders schwierigen Fällen, beispielsweise nach mehrmaligen Reoperationen, lässt sich die Verankerung der Pfanne zusätzlich verbessern, wenn am basisseitigen Rand der Aussenschale mit Durchtrittsöffnungen versehene, sich radial oder peripher erstreckende Laschen angeordnet sind.

Im folgenden wird die Erfindung anhand von Ausführungsbeispielen im Zusammenhang mit der Zeichnung näher erläutert.

Fig. 1 ist eine Ansicht vom Aequator in Richtung zum Pfannenscheitel in die Aussenschale hinein;

Fig. 2 gibt eine Ansicht von Fig. 1 von der Seite;

Fig. 3 ist eine Ansicht von Fig. 2 von oben;

Fig. 4 stellt die gleiche Ansicht wie Fig. 3 für den Kunststoff-Pfannenkörper dar;

Fig. 5 zeigt schematisch teilweise im Schnitt eine Ausführungsform der neuen Pfanne, eingesetzt in einen Beckenknochen;

Fig. 6 ist ein vergrösserter Ausschnitt der Aussenschale von Fig. 5;

Fig. 7 zeigt einen, in den Dübeln nach Fig. 6 einschlagbaren Bolzen, während die

Fig. 8, 9, 10 verschiedene Ausführungsformen der Aussen schale in gleicher Darstellung und Blickrichtung wie Fig. 1 wiedergeben.

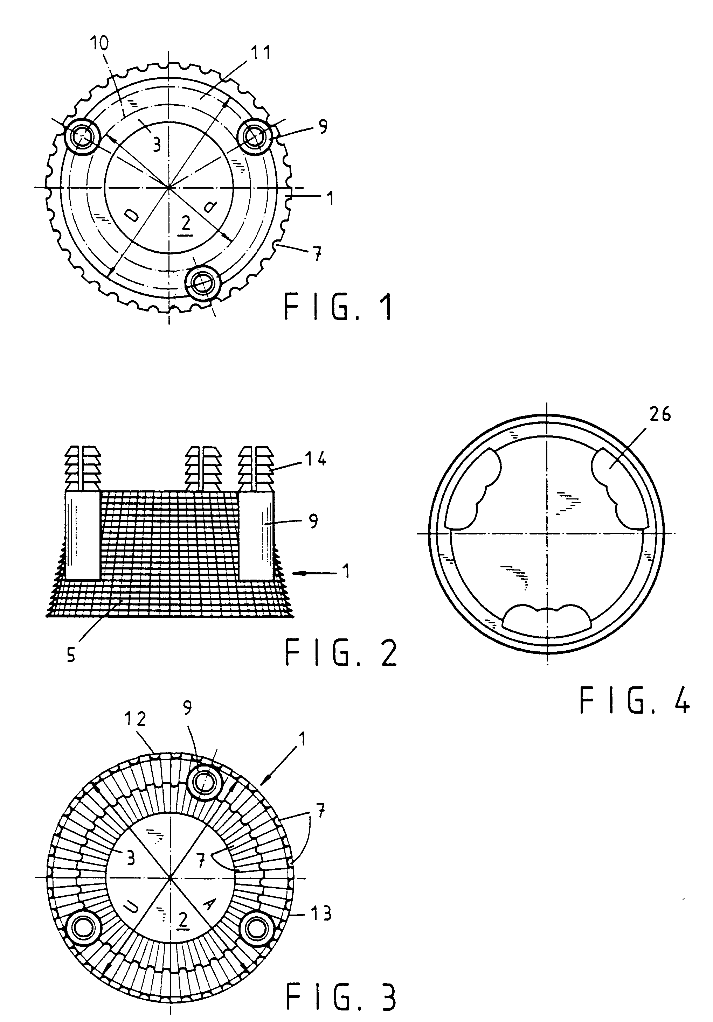

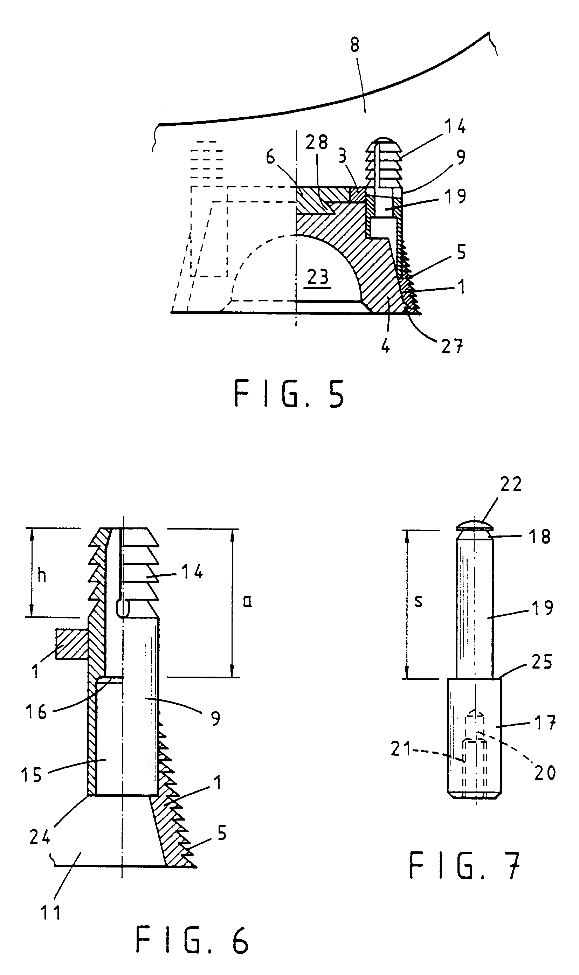

Die aus Metall, vorzugsweise aus Titan oder einer Titanlegierung, bestehende Aussenschale 1 der neuen Hüftgelenkspfanne hat die Form eines Kegelstumpfes, dessen "kopfseitige" Grundfläche 3 (Fig. 3) eine mit ihm konzentrische kreisförmige Oeffnung 2 aufweist. Der Flächenanteil an der zugehörigen Grundfläche 3 beträgt etwa 45 %. Die Oeffnung 2 ist durch einen Metalldeckel 6 (Fig. 5) verschliessbar und dient zum Hinterfüllen der implantierten Aussenschale 1 mit Knochenmaterial. Durch den Deckel 6 soll dabei jeglicher Kontakt von lebendem Gewebe mit dem Kunststoff-Pfannenkörper 4 (Fig. 5) verhindert werden. Die Form des Kegelstumpfes ermöglicht eine einfache Zentrierung der Pfanne beim Implantieren und erleichtert das zentrierte Ansetzen von Bohrlehren und Fräsern bei der operativen Vorbereitung des Knochens.

Die äussere Mantelfläche der Aussenschale 1 ist mit einer sägezahnartigen - d.h. mit einer schrägen und einer horizontalen Flanke ausgestatteten - zirkulären Feinverzahnung 5 versehen, deren Zahntiefe im Maximum 1 mm beträgt, um zu gewährleisten, dass sich zwischen den Zähnen 5 spongioses Knochengewebe - und nicht nur zu einer festen Verankerung kaum beitragendes Bindegewebe - bildet.

In der Verzahnung 5 der Mantelfläche können ebenso wie in der kopfseitigen Grundfläche 3 entlang Mantellinien bzw. entlang Flächenradien verlaufende Rillen 7 vorhanden sein, die die Oberfläche der Aussenschale 1 vergrössern, wodurch bekanntlich die Haftung zwischen knochen 8 (Fig. 5) und Implantat 1 verbessert wird.

Für eine primäre Fixierung der Aussenschale 1 im knochen 8 ragen aus deren Mantelfläche mindestens drei als Spreizdübel 9 ausgebildete Zapfen hervor, die bezüglich einer Rotation um die Kegelstumpfachse unsymmetrisch auf dem Umfang der Aussenschale 1 verteilt sind, so dass für die Aussenschale 1 nur eine einzige definierte Einbaustellung gegeben ist.

Die Spreizdübel 9 sind dabei möglichst weit an den Rand der Aussenschale 1 gesetzt, um zwischen ihn eine grosse "Verankerungsfläche" zu erhalten und die Wahrscheinlichkeit dafür, dass sie sich in gesundem Knochengewebe erstrecken, zu erhöhen. Die "Dicke" der Spreizdübel 9 ist relativ zur Grösse der Aussenschale so gewählt, dass einerseits der Durchmesser d eines die Dübel 9 berührenden Innenkreises 10 (Fig. 1) mindestens 2/3 des basisseitigen Innendurchmessers D des Innenhohlraumes 11 der Aussenschale 1 beträgt, und dass andererseits der Durchmesser U (Fig. 3) des die Dübel umhüllenden Aussenkreises 12 kleiner ist als der Aussendurchmesser A des basisseitigen Aussenrandes 13 der Aussenschale 1. Die zuletzt genannte Bemessung stellt sicher, dass die Aussenbegrenzung der operativ zu schaffenden Knochenausfräsung als einfacher Kegelstumpf ausgeführt werden kann und nicht mit den Rand "überragenden" Ausbuchtungen versehen werden muss.

Weiterhin ist die geschlitzte, spreizbare Höhe h (Fig. 6) eines Dübels 9 auf seinen den Kegelstumpf 1 überragenden Bereich beschränkt und mit einer sägezahnartigen Verzahnung 14 versehen, deren Zahntiefe bis zu 3 mm betragen kann. Innerhalb des Kegelstumpfes 1 ist die Oberfläche des Dübels 9 verzahnungsfrei, um ein geführtes Einschieben der Aussenschale in entsprechende Bohrungen im Beckenknochen zu erleichtern; er kann jedoch, beispielsweise durch Sandstrahlen, aufgerauht sein.

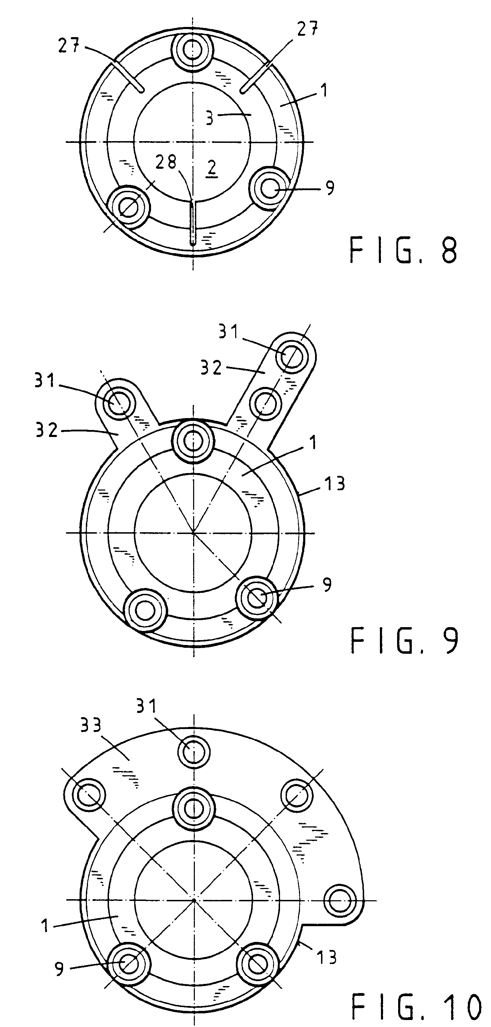

Wie Fig. 6 verdeutlicht, ist die Innenbohrung 15 eines Dübels 9 mit einer Absatz 16 versehen. Dessen Abstand a vom freien Ende des Dübels ist auf eine Länge s zwischen dem durch eine Schulter 25 abgesetzten Kopf 17 und einer Kerbe 18 am freien Ende eines als Spreizelement dienenden Bolzens 19 (Fig. 7) angeglichen. In die Kerbe 18 des Bolzens 19, der in Richtung der Dübelachse ohne Schraubbewegung in den Dübel 9 eingeschlagen wird, rastet nach dem Einschlagen das freie Ende des Dübels 9 ein und bewirkt eine Sicherung gegen unbeabsichtigtes Zurückgleiten des Bolzens 19. Weiterhin wird dadurch erreicht, dass in jedem Fall die Spreizkraft in den Dübel 19 gleich und unabhängig von dem Kraftaufwand des implantierenden Operateurs ist. Für ein Ansetzen eines Ausziehinstrumentes zum gewollten Entfernen des Bolzens 19 ist im Kopf 17 eine Sackbohrung 20 mit einem Gewinde 21 vorgesehen. Bei einem solchen gewollten Entfernen des Bolzens wird dessen pilzkopfartiges Ende 22 abgerissen.

Der Kunststoff-Pfannenkörper 4, der die eigentliche Pfannenschale 23 für die Aufnahme eines nicht gezeigten Gelenkkopfes einer Femurkopfprothese enthält, ist mit seiner Aussenform an den Innenhohlraum 11 der Aussenschale 1 angepasst. Um bei der Verwendung von - beispielsweise durch eine nicht gezeigte einseitige Pfannendacherhöhungnicht rotationssymmetrischen Pfannenkörpern 4 eine gewisse "Einstellkorrektur" für die Unsymmetrie relativ zur Aussenschale 1 zu ermöglichen, hat die Aussenform des Pfannenkörpers 4 für jeden Dübel 9, dessen Kontur 24 (Fig. 6) teilweise in den Innenhohlraum 11 (Fig. 6) der Aussenschale 1 hinein- ragt, mehrere mondsichelartige Ausdrehnungen 26 (Fig. 4), deren Mittelpunkte jeweils um 15° versetzt zueinander sind.

Mit je einem Schnappverschluss 27 und 28 sind der Pfannenkörper 4 in der Aussenschale 1 und der Deckel 6 auf dem Pfannenkörper 4 gehalten.

Die Elastizität der Aussenschale 1 für ein dynamisches elastisches radiales Aufweiten und Zusammenziehen des Beckens kann verbessert werden, wenn in die Aussenschale 1 in Meridianebenen angeordnete Schlitze 27 und 28 von beiden Stirnflächen des Kegelstumpfes 1 her eingeschnitten sind (Fig. 8).

Eine bevorzugte Anwendung der neuen Hüftgelenkspfanne ist das Gebiet der Reoperationen. Bei diesen ist die für die Verankerung der neuen einzusetzenden Pfanne zur Verfügung stehende Knochensubstanz häufig gering. Um trotzdem eine sichere Verankerung zu gewährleisten, ist es möglich, am äquatorialen Aussenrand 13 der Aussenschale 1 mit Durchtrittsöffnungen 31 versehene Laschen 32 und 33 anzubringen, mit denen die Pfanne durch Spreizdübel oder Knochenschrauben zusätzlich im Becken fixiert werden kann (Fig. 9 und 10). Die Laschen 32 und 33 können dabei relativ zur Aussenschale 1 radial und/oder peripher verlaufen; darüberhinaus können sie intraoperativ verformbar ausgebildet sein.

## Patentansprüche

1. Künstliche Hüftgelenkspfanne für eine zementfreie Verankerung im Beckenknochen, welche Pfanne aus einer metallischen Aussenschale (1) und einem KunststoffPfannenkörper (4) besteht, der die eigentliche Pfannen schale zur Aufnahme eines Gelenkkopfes enthält, wobei die Aussenschale (1) die Form eines Kegelstumpfes hat, dessen Grundfläche (3) im Scheitelbereich mindestens eine Oeffnung (2) aufweist, wobei ferner die äussere Oberfläche der Aussenschale (1) mit einer zirkulären Verzahnung versehen ist, <u>gekennzeichnet durch die Kombination folgender Merkmale:</u>
  – Die Verzahnung (5) ist sägezahnartig ausgebildet und weist eine Zahntiefe von maximal 1 mm auf;
  – aus dem Mantel des Kegelstumpfes der Aussenschale (1) ragen mindestens drei, ungleichmässig auf dem Umfang verteilte, als Spreizdübel (9) ausgebildete Veranke rungszapfen hervor, deren Achsen parallel zur Kegel stumpfachse verlaufen;
  – die geschlitzte, spreizbare Höhe (h) der Dübel (9) ist auf ihren den Kegelstumpf (1) überragenden Abschnitt beschränkt und trägt ebenfalls eine sägezahnartige, zirkuläre Verzahnung (14), während die Dübeloberfläche im Bereich der Kegelstumpfhöhe verzahnungsfrei ausgebildet ist;
  – der Durchmesser (d) eines die Dübel (9) berührenden, mit dem Kegelstumpf (1) konzentrischen Innenkreises (10) beträgt mindestens 2/3 des basisseitigen Innendurchmessers (D) des den Pfannenkörper (4) aufnehmenden Innenhohlraumes (11) der Aussenschale (1), während der Durchmesser (U) des zugehörigen, die Dübel (9) umschliessenden Aussenkreises (12) kleiner ist als derjenige (A) des basisseitigen Aussenrandes (13) der Aussenschale (1).

2. Hüftgelenkspfanne nach Anspruch 1, **dadurch gekennzeichnet, dass** die Verzahnung (5) der äusseren Oberfläche der Aussenschale (1) mit entlang Mantellinien und Radien verlaufenden Rillen (7) versehen ist.

3. Hüftgelenkspfanne nach Anspruch 1 oder 2, **dadurch gekennzeichnet, dass** als Spreizelemente gewindefreie, in Längsrichtung eines Dübels (9) einschlagbare, zylindrische Bolzen (19) dienen, die an einem Ende mit einem durch eine Schulter (25) abgesetzten Kopf (17) und am anderen Ende mit einer durch eine zirkuläre Führungsrille erzeugten Kerbe (18) ausgestattet sind.

4. Hüftgelenkspfanne nach Anspruch 3, **dadurch gekennzeichnet, dass** der Abstand (s) zwischen der Schulter (25) und der Kerbe (18) des Spreizelementes (19) gleich demjenigen (a) eines Absatzes (16) für die Auflage des Kopfes(17) im Innenhohlraum(15) des Dübels (9) und dessem freien Ende ist.

5. Hüftgelenkspfanne nach einem der Ansprüche 1 bis 4, **dadurch gekennzeichnet, dass** die Fläche der Öffnung (2) im Scheitelbereich der Aussenschale (1) mindestens 1/3 der Grundfläche des Kegelstumpf-Kopfkreises beträgt und durch einen metallischen Deckel (6) verschliessbar ist.

6. Hüftgelenkspfanne nach einem der Ansprüche 1 bis 5, **dadurch gekennzeichnet, dass** die Aussenschale (1) von ihren beiden Rändern her mit Einschnitten (27, 28) versehen ist.

7. Hüftgelenkspfanne nach einem der Asprüche 1 bis 6, **dadurch gekennzeichnet, dass** am basisseitigen Aussenrand (13) der Aussenschale (1) mit Durchtrittsöffnungen (31) versehene, sich radial oder peripher erstreckende Laschen (32, 33) angeordnet sind.

## Claims

1. An acetabular cup prosthesis for uncemented fixing in the pelvis, the prosthesis comprising a metal outer shell (1) and a plastics socket (4), the latter containing the actual shell for receiving a femoral head, the outer shell being in the shape of a frustum whose base surface (3) is formed with at least one aperture (2) in the apex zone, the outer surface of the outer shell (1) having circular toothing, characterised by the combination of the following features:
   – The toothing (5) is sawtoothed and has a tooth depth of at most 1 mm;
   – At least three fixing pins whose axes extend parallel to the frustum axis and which are in the form of expanding dowels (9) and which are distributed irregularly over the periphery project from the generated surface of the frustum of the outer shell (1);
   – The slotted expandable height (h) of the dowels (9) is limited to their part extending beyond the frustum (1) and also has sawtooth circular toothing (14), the dowel surface being untoothed near the height of the frustum;
   – The diameter (d) of an inner circle (10) which contacts the dowels (9) and is concentric of the frustum (1) is at least two-thirds of the base inner diameter (D) of the socket-receiving interior (11) of the outer shell (1) while the diameter (U) of the associated outer circle (12) which extends around the dowels (9) is less than the diameter (A) of the base outer edge (13) of the outer shell (1).

2. A prosthesis according to claim 1, characterised in that the toothing (5) of the outer surface of the outer shell (1) is formed with grooves (7) which extend along generatrices and radii.

3. A prosthesis according to claim 1 or 2, characterised in that non-screwthreaded cylindrical pins introducible lengthwise of a dowel (9) are used as expanding elements, have at one end a shouldered (25) head (17) and are formed at the other end with a recess (18) arising from a circular guide groove.

4. A prosthesis according to claim 3, characterised in that the distance (s) between the shoulder (25) and the recess (18) is equal to the distance (a) between a shoulder (16) serving as support surface for the head (17) in the dowel interior (15) and the free end of the dowel (9).

5. A prosthesis according to any of claims 1 to 4, characterised in that the area of the aperture (2) in the apex zone of the outer shell (1) is at least one-third of the base surface of the tip circle of the frustum and is closable by a metal cover (6).

6. A prosthesis according to any of claims 1 to 5, characterised in that the outer shell (1) is formed with incisions (27, 24) which extend from both its edges.

7. A prosthesis according to any of claims 1 to 6, characterised in that tabs (32, 33) which are formed with through apertures (31) and which extend radially or peripherally are disposed on the base-side outer edge (13) of the outer shell (1)

## Revendications

1. Cavité cotyloïde artificielle pour un ancrage sans ciment dans l'os du bassin, laquelle cavité est constituée d'une coque extérieure (1) métallique et d'un corps de cavité (4) en matière plastique qui contient la coque de cavité proprement dite destinée à loger une tête d'articulation, la coque extérieure (1) ayant la forme d'un tronc de cône dont la surface de base (3) présente au moins une ouverture (2) dans la région du sommet, la surface extérieure de la coque extérieure (1) étant en outre pourvue d'une denture circulaire, caractérisée par la combinaison des caractéristiques suivantes :
   – la denture (5) est en dents de scie et présente une profondeur de dent de 1 mm au maximum ;

– de l'enveloppe du tronc de cône de la coque extérieure (1) font saillie au moins trois pivots d'ancrage, conçus sous la forme de chevilles à expansion (9), uniformément répartis sur le pourtour, dont les axes sont parallèles à l'axe du tronc de cône ;

– la hauteur (h) d'expansion, fendue, des chevilles (9) est limitée sur leur partie dépassant du tronc de cône (1) et porte aussi une denture (14) circulaire, en dents de scie, tandis que la surface de la cheville ne présente pas de dents dans la région de la hauteur du tronc de cône ;

– le diamètre (d) d'un cercle intérieur (10) en contact avec les chevilles (9), concentrique au tronc de cône (1), est au moins égal à 2/3 du diamètre intérieur (D) côté base de la cavité intérieure (11) de la coque extérieure (1), logeant le corps de cavité (4), tandis que le diamètre (U) du cercle extérieur (12) correspondant, entourant les chevilles (9), est inférieur au diamètre (A) du bord extérieur (13) côté base de la coque extérieure (1).

2. Cavité cotyloïde selon la revendication 1, caractérisée en ce que la denture (5) de la surface extérieure de la coque extérieure (1) est pourvue de gorges (7) s'étendant le long de génératrices et de rayons.

3. Cavité cotyloïde selon la revendication 1 ou 2, caractérisée en ce qu'on utilise comme éléments à expansion des goujons (19) cylindriques sans filetage, à enfoncer dans la direction longitudinale d'une cheville (9), lesquels goujons sont pourvus à une extrémité d'une tête (17) étagée par un épaulement (25) et, à l'autre extrémité, d'une encoche (18) produite par une gorge de guidage circulaire.

4. Cavité cotyloïde selon la revendication 3, caractérisée en ce que la distance (s) séparant l'épaulement (25) de l'encoche (18) de l'élément à expansion (19) est égale à la distance (a) séparant un gradin (16), servant d'appui à la tête (17) dans la cavité intérieure (15) de la cheville (9), de son extrémité libre.

5. Cavité cotyloïde selon l'une des revendications 1 à 4, caractérisée en ce que la surface de l'ouverture (2) dans la région du sommet de la coque extérieure (1) est au moins égale à 1/3 de la surface de base du cercle de tête du tronc de cône et peut être fermée par un couvercle (6) métallique.

6. Cavité cotyloïde selon l'une des revendications 1 à 5, caractérisée en ce que la coque extérieure (1) est pourvue d'entailles (27, 28), à partir de ses deux bords.

7. Cavité cotyloïde selon l'une des revendications 1 à 6, caractérisée en ce que sur le bord extérieur (13) côté base de la coque extérieure (1) sont disposées des pattes (32, 33) s'étendant radialement ou périphériquement et pourvues d'ouvertures de passage (31).

FIG. 1

FIG. 2

FIG. 4

FIG. 3

FIG. 5

FIG. 6

FIG. 7

FIG. 8

FIG. 9

FIG. 10